# EUROPEAN PATENT SPECIFICATION

(11) **EP 1 145 724 B1**
(45) Date of publication and mention of the grant of the patent: **14.04.2004**
(21) Application number: 00303141.6
(22) Date of filing: 13.04.2000
(51) Int. Cl.: A61L 15/60, A61L 15/18, A61L 28/00

(54) **Absorbent material and method of production**
Absorbierendes Material und Verfahren zu seiner Herstellung
Matériau absorbant et procédé de production de ce materiau

(43) Date of publication of application: 17.10.2001
(73) Proprietor: BRISTOL-MYERS SQUIBB COMPANY, Princeton, NJ 08543-4000 (US)
(72) Inventor: Troy, Padraig Gerard, Crawley, W. Sussex RH10 5DL (GB); Steer, Graham Emery, Wimbledon, London SW20 0TB (GB)
(74) Representative: Holmes, Miles Keeton

(56) References cited:
- EP-A- 0 138 427
- EP-A- 0 629 411
- EP-A- 0 891 758
- GB-A- 2 301 350
- GB-A- 2 312 213
- US-A- 5 489 469

## Description

This invention relates to an absorbent material, and to a method of producing the material. The invention is especially suitable for absorbing liquid matter excreted by the body (e.g. for use in ostomy, incontinence or woundcare), and/or for absorbing aqueous matter, but it is not limited exclusively to these fields.

GB-A-2301350 and EP-A-0891758 describe superabsorbent formulations and methods of producing articles containing superabsorbent material. A significant aspect of the superabsorbent formulation is the inclusion of glycerol and water, which binds the superabsorbent powder granules together when compressed, to form a consolidated mass. The superabsorbent material can be formed into tablets, or compressed between upper and lower layers to form a sheet-like product.

However, there are practical limits on the amount of glycerol and water which the superabsorbent is able to take up as a consolidatable mass. This limits the structural stability of the consolidated mass, and makes it vulnerable to at least some degree of shedding (particularly at the edges). Also, articles made of the material tend not to be flexible, and may shed material if bent or flexed.

Reference may also be made to EP 0629411 (relative to which the invention is characterised).

It would be desirable to provide a way of increasing the amount of glycerol and water which can be incorporated in the formulation without detracting significantly from other advantageous characteristics of the superabsorbent formulation.

The present invention is defined in the claims.

The preferred embodiment illustrates including silica in a formulation including superabsorbent material (e.g. powder or granules). This has been found to provide the following advantages:
(a) the silica acts as a lubricant, enabling the superabsorbent particles to flow more easily, thus making handling of the superabsorbent particles easier, and leading to easier mixing and compressing processes; and
(b) the silica also enables more glycerol/water to be taken up by the superabsorbent material. It is believed that the silica enables the glycerol/water to penetrate between the particles of superabsorbent material more completely, thereby increasing the quantity of glycerol/water which can be taken up.

The amount of silica present (per 100 parts wt of superabsorbent material) may be at least 6 parts, more preferably at least 7 parts, and in one form at least 8 parts.

Preferably, the amount of silica present (per 100 parts wt of superabsorbent material) is not greater than 20 parts, more preferably not greater than 15 parts, more preferably not greater than 10 parts and, in one form, not greater than 8 parts.

The optimum amount of silica depends to some extent on the size of the particles of superabsorbent. Generally, it is preferred that the silica be present in such a quantity to provide a surface coating on substantially all of the superabsorbent particles. Preferably, each particle, is substantially entirely coated. It will be appreciated that a smaller particle size results in a larger surface area to be coated per unit volume, thus requiring a greater quantity of silica for optimum effect.

The superabsorbent particles may comprise an alkali metal polyacrylate (e.g. sodium polyacrylate), or some other polyacrylate, or other material which provides superabsorbent properties (defined generally as the ability to absorb many times their own weight of liquid matter). Suitable materials include for example, water-swellable polymers.

Preferably, the formulation includes glycerol. Alternatively, another non-volatile lubricious polyhydroxy compound may be used which is water-soluble or water-dispersible at or below 40°C and is liquid at room temperature (15 to 20°C). Suitable other compounds include, for example: propane 1-2 diol; polyethylene glycol 200 (or 300); or sorbitol.

Preferably, the amount of glycerol (or other material) is 5-40 parts per 100 parts of the superabsorbent material, more preferably 5-30 parts, more preferably 10-25 parts. In one embodiment, the amount of glycerol (or other material) is between 12 and 18 parts per 100 parts of superabsorbent.

The formulation also includes water. Preferably, the water is present in an amount of 0.5 to 10 parts by weight per 100 parts of superabsorbent, more preferably 0.5 to 6 parts. In one example, the amount of water is between 3 and 5 parts.

The formulation may also include additives, for example, a malodour counteractant.

The preferred embodiment also illustrates a method of producing an absorbent material, including:
(a) providing superabsorbent particles which are substantially entirely coated with silica; and
(b) mixing with the silica coated superabsorbent, water and a non-volatile lubricious polyhydroxy compound which is water-soluble or water-dispersible at or below 40°C and is liquid at at least one temperature in the range of 15-20°C.

An embodiment of the invention is now described by way of example only, with reference to the accompanying drawings, in which:
Fig. 1 is a schematic flow diagram showing a production process for producing an absorbent material;
Figs. 2a and 2b are schematic drawings showing the superabsorbent granules before (a) and after (b) mixing with silica; and
Fig. 3 is a schematic drawing of apparatus for consolidating the material to form shaped articles.

An example superabsorbent formulation comprises, in parts by weight (pbw):

| | |
|---|---|
| Superabsorbent | 100 pbw |
| Silica | 6-10 pbw |
| Glycerol | 14 pbw |
| Water | 4 pbw. |

A suitable superabsorbent material is, for example, sodium polyacrylate available under trade name FemDry 33C from Chemdal. Sodium polyacrylate superabsorbent materials are also available from Dow Chemicals and from Hoescht.

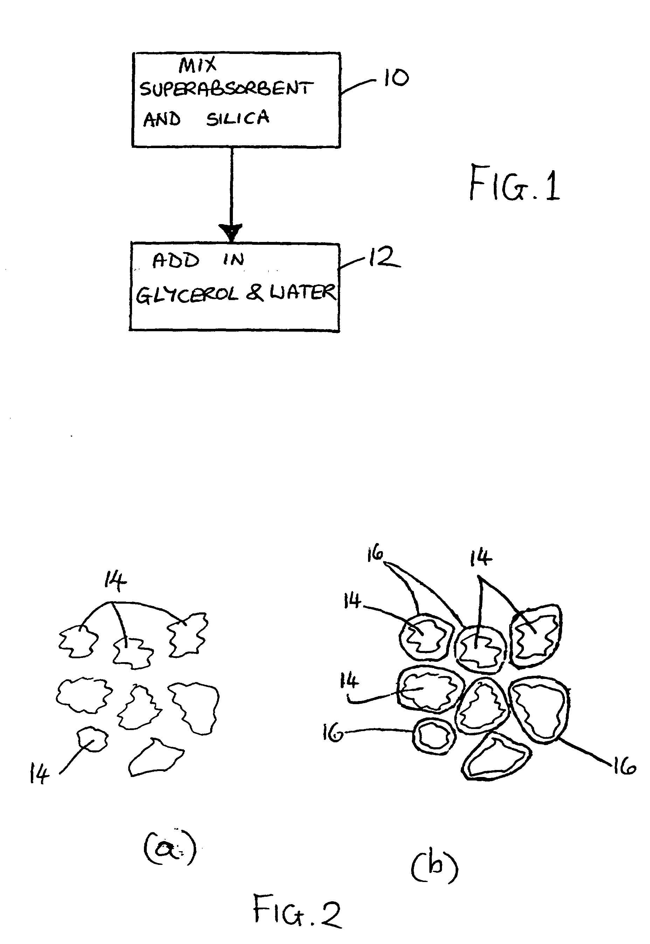

Referring to Figs. 1 and 2, the formulation is prepared in first and second stages 10 and 12. The first stage is to mix together the superabsorbent granules and the silica. Fig. 2a shows the superabsorbent granules 14 prior to mixing, and Fig. 2b shows the granules 14 after mixing. As can be seen from Fig. 2b, the effect of mixing is to coat the granules 14 with a thin coating of silica 16. The silica 16 acts as a lubricant making the granules easier to flow, and therefore easier to mix.

The second stage 12 is to add a premix of glycerol and water to the silica coated superabsorbent formulation. The effect of the silica is generally to enable the superabsorbent formulation to take up a greater quantity of glycerol/water than if the silica were absent. It is believed that this is as a result of the silica (again acting as a form of lubricant) permitting the glycerol/water to penetrate more completely between the superabsorbent granules 14. The optimum quantity of silica depends at least to some extent on the size of the superabsorbent granules 14. It is preferred that the quantity of silica be sufficient to coat substantially the entire surface of the superabsorbent particles 14. During the second stage 12, the formulation is mixed to a powder consistency.

The superabsorbent formulation can be formed into a desired shaped article by compression, which consolidates the powder structurally, in the same manner as that described in GB-A-2301350 and EP-A-0891758 referred to hereinbefore. The presence of the glycerol/water (particularly in the higher quantity than that attainable hitherto) enables the mixture to be compressed at room temperature with only relatively mild pressure being required. In most cases no other heat is required. Moreover, the increased quantity of glycerol/water provides greater structural stability than that achievable hitherto, so that the formed article is less prone to shedding, and can be at least partly flexible if desired.

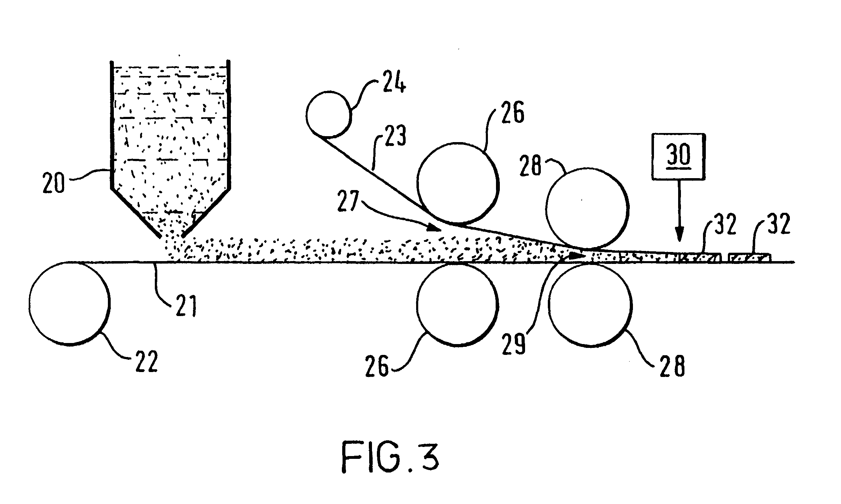

Fig. 3 illustrates, by way of example, apparatus 18 for forming a sheet of superabsorbent, suitable for use in ostomy, incontinence or woundcare applications. The powder formulation is fed from a supply 20 onto a travelling sheet 21 of tissue paper which is pulled off a supply roll 22. A second (overhead) sheet 23 of tissue paper is fed from a second supply roll 24 and these two sheets sandwich the powder between them. The product passes between a first pair of rolls 26 which form a first nip 27 and to a second pair of rolls 28 forming a second nip 29. The inter-roll spacing at the second nip 29 may be for example 0.1 to 2.0 mm. That at the first nip 27 may be 0.75 to 4 mm. No external heat is applied. The resulting product is a flat sheet which can then be cut into suitable shapes, e.g. rectangles 32, and can be directly placed in an ostomy pouch or incontinence pouch. It has been found to rapidly absorb a liquid such as urine. In tests, over 60 ml of synthetic urine were absorbed in under 60 seconds, usually under 40 seconds, by a product of one square centimetre area and 2 mm thickness made by the method described above.

If desired, the sheet may be processed during production to form packet-like articles, as described in EP-A-0891758.

Reference has been made in the above specification to malodour counteractants. One example of a malodour counteractant is a bactericide, e.g. benzyl alkonium chloride. Another example is a fragrance. Other examples are included in a range of bactericides manufactured by ConvaTec™ (Calgon-Vestal Div.). Yet other malodour counteractants which may be used are methylchlorosiothiazolinone, methylisothiazolinone, or denatonium benzoate.

It will be appreciated that the foregoing description is merely illustrative of preferred forms of the invention, and that many modification may be made within the scope of the invention. Aspects believed to be of particular importance are identified in the claims. However, the applicant claims protection for any novel feature or idea described and/or illustrated herein whether or not emphasis has been placed thereon.

## Claims

1. A formulation for utilisation to absorb liquid, comprising:
superabsorbent;
silica;
water; and
a non-volatile lubricious polyhydroxy compound which is water-soluble or water-dispersible at or below 40°C and is liquid at at least one temperature within the range 15 to 20°C,
**characterised in that** the relative quantity of the silica is at least 6 parts by weight per 100 parts by weight of the superabsorbent.

2. A formulation according to claim 1, wherein the relative quantities in the formulation are, in parts by weight (pbw):
| | |
|---|---|
| superabsorbent | 100 pbw |
| silica | 6-20 pbw |
| water | 0.5 - 10 pbw |
| said lubricious polyhydroxy compound | 5-40 pbw. |

3. A formulation according to claim 1 or 2, wherein the silica is present in a quantity to provide a surface coating on at least the majority of the surface area of the superabsorbent.

4. A formulation according to claim 3, wherein the silica is present in a quantity to provide a surface coating on substantially the entire surface area of the superabsorbent.

5. A formulation according to any preceding claim, wherein the silica is present in a quantity of at least 7 pbw.

6. A formulation according to claim 5, wherein the silica is present in a quantity of at least 8 pbw.

7. A formulation according to any preceding claim wherein the polyhydroxy compound is glycerol.

8. A formulation according to any preceding claim, wherein the polyhydroxy compound is present in an amount of 5-30 pbw.

9. A formulation according to any preceding claim, wherein the polyhydroxy compound is present in an amount of at least 12 pbw.

10. A formulation according to claim 9, wherein the polyhydroxy compound is present in an amount of at least 13 pbw.

11. A formulation according to claim 10, wherein the polyhydroxy compound is present in an amount of at least 14 pbw.

12. A formulation according to any preceding claim, wherein the water is present in an amount of 0.5-6 pbw.

13. A formulation according to any preceding claim, wherein the water is present in an amount of at least 3 pbw.

14. A formulation according to claim 13, wherein the water is present in an amount of at least 4 pbw.

15. A formulation according to any preceding claim, wherein the superabsorbent comprises a water swellable polymer.

16. A formulation according to claim 15, wherein the superabsorbent comprises a polyacrylate.

17. A formulation according to claim 16, wherein the polyacrylate is an alkali metal polyacrylate.

18. A formulation according to claim 17, wherein the superabsorbent comprises sodium polyacrylate.

19. A formulation according to any preceding claim, wherein the formulation is consolidatable upon compression to form a consolidated mass.

20. A method of producing a superabsorbent formulation, the method **characterised by**:
(a) providing superabsorbent particles which are substantially entirety coated with silica in an amount of at least 6 parts by weight of silica per 100 parts by weight of the superabsorbent; and
(b) mixing with the silica coated superabsorbent, water and a non-volatile lubricious polyhydroxy compound which is water-soluble or water-dispersible at or below 40°C and is liquid at at least one temperature in the range of 15°C-20°C.

21. A method according to claim 20, wherein the step (a) comprises the sub-step of:
mixing together superabsorbent and silica to coat the superabsorbent with silica.

22. A method according to claim 20 or 21, wherein the lubricious polyhydroxy compound is glycerol.

23. A method according to claim 20, 21 or 22, wherein the relative quantities in the formulation are, in parts by weight (pbw):
| | |
|---|---|
| superabsorbent | 100 pbw |
| silica | 6-20 pbw |
| water | 0.5-10 pbw |
| said lubricious polyhydroxy compound | 5-40 pbw. |

24. A liquid absorbent article comprising a formulation as defined in any of claims 1 to 21.

25. An article according to claim 24, wherein the article comprises a consolidated mass including said formulation.

26. An ostomy, incontinence or woundcare product comprising or containing a superabsorbent formulation as defined in any of claims 1 to 19.

27. A product according to claim 26, wherein the product is an ostomy pouch.

28. Use of a superabsorbent formulation as defined in any of claims 1 to 19 in an ostomy, incontinence or woundcare product.

29. Use according to claim 28, wherein said formulation is used in a consolidated form as a liquid absorbent article.

## Patentansprüche

1. Folgendes aufweisende Zubereitung zur Verwendung zum Absorbieren von Flüssigkeit:
Superabsorbens;
Siliciumdioxid;
Wasser und
eine nichtflüchtige schmierende Polyhydroxyverbindung, die bei 40 °C oder darunter wasserlöslich oder wasserdispergierbar ist und bei mindestens einer Temperatur in dem Bereich von 15 bis 20 °C flüssig ist,
**dadurch gekennzeichnet, dass** die relative Menge des Siliciumdioxids mindestens 6 Masseteile auf 100 Masseteile des Superabsorbens beträgt.

2. Zubereitung nach Anspruch 1, **dadurch gekennzeichnet, dass** die relativen Mengen, in Masseteilen, in der Zubereitung
| | |
|---|---|
| für das Superabsorbens | 100 Masseteile, |
| für das Siliciumdioxid | 6 - 20 Masseteile, |
| für das Wasser | 0,5 - 10 Masseteile, |
| für die schmierende Polyhydroxyverbindung | 5 - 40 Masseteile |
betragen.

3. Zubereitung nach Anspruch 1 oder 2, **dadurch gekennzeichnet, dass** das Siliciumdioxid in einer solchen Menge vorliegt, dass es eine Oberflächenschicht auf mindestens dem größten Teil der Oberfläche des Superabsorbens liefert.

4. Zubereitung nach Anspruch 3, **dadurch gekennzeichnet, dass** das Siliciumdioxid in einer solchen Menge vorliegt, dass es eine Oberflächenschicht auf im Wesentlichen der gesamten Oberfläche des Superabsorbens liefert.

5. Zubereitung nach einem vorhergehenden Anspruch, **dadurch gekennzeichnet, dass** das Siliciumdioxid in einer Menge von mindestens 7 Masseteilen vorliegt.

6. Zubereitung nach Anspruch 5, **dadurch gekennzeichnet, dass** das Siliciumdioxid in einer Menge von mindestens 8 Masseteilen vorliegt.

7. Zubereitung nach einem vorhergehenden Anspruch, **dadurch gekennzeichnet, dass** die Polyhydroxyverbindung Glycerol ist.

8. Zubereitung nach einem vorhergehenden Anspruch, **dadurch gekennzeichnet, dass** die Polyhydroxyverbindung in einer Menge von 5 - 30 Masseteilen vorliegt.

9. Zubereitung nach einem vorhergehenden Anspruch, **dadurch gekennzeichnet, dass** die Polyhydroxyverbindung in einer Menge von mindestens 12 Masseteilen vorliegt.

10. Zubereitung nach Anspruch 9, **dadurch gekennzeichnet, dass** die Polyhydroxyverbindung in einer Menge von mindestens 13 Masseteilen vorliegt.

11. Zubereitung nach Anspruch 10, **dadurch gekennzeichnet, dass** die Polyhydroxyverbindung in einer Menge von mindestens 14 Masseteilen vorliegt.

12. Zubereitung nach einem vorhergehenden Anspruch, **dadurch gekennzeichnet, dass** das Wasser in einer Menge von 0,5 - 6 Masseteilen vorliegt.

13. Zubereitung nach einem vorhergehenden Anspruch, **dadurch gekennzeichnet, dass** das Wasser in einer Menge von mindestens 3 Masseteilen vorliegt.

14. Zubereitung nach Anspruch 13, **dadurch gekennzeichnet, dass** das Wasser in einer Menge von mindestens 4 Masseteilen vorliegt.

15. Zubereitung nach einem vorhergehenden Anspruch, **dadurch gekennzeichnet, dass** das Superabsorbens ein in Wasser quellbares Polymer aufweist.

16. Zubereitung nach Anspruch 15, **dadurch gekennzeichnet, dass** das Superabsorbens ein Polyacrylat aufweist.

17. Zubereitung nach Anspruch 16, **dadurch gekennzeichnet, dass** das Polyacrylate in Alkalimetall-Polyacrylat ist.

18. Zubereitung nach Anspruch 17, **dadurch gekennzeichnet, dass** das Superabsorbens Natriumpolyacrylat aufweist.

19. Zubereitung nach einem vorhergehenden Anspruch, **dadurch gekennzeichnet, dass** die Zubereitung bei Komprimierung verdichtet werden kann, um eine verdichtete Masse herzustellen.

20. Verfahren zur Herstellung einer Superabsorbens-Zubereitung, das durch folgende Schritte **gekennzeichnet** ist:
(a) Bereitstellen von Superabsorbens-Teilchen, die im Wesentlichen vollständig mit Siliciumdioxid in einer Menge von mindestens 6 Masseteilen Siliciumdioxid auf 100 Masseteile des Superabsorbens beschichtet sind, und
(b) Mischen des Siliciumdioxid-beschichteten Superabsorbens mit Wasser und einer nichtflüchtigen schmierenden Polyhydroxyverbindung, die bei 40 °C oder darunter wasserlöslich oder wasserdispergierbar ist und bei mindestens einer Temperatur in dem Bereich von 15 bis 20 °C flüssig ist.

21. Verfahren nach Anspruch 20, **dadurch gekennzeichnet, dass** der Schritt (a) den Teilschritt des Vermischens des Superabsorbens mit dem Siliciumdioxid, um das Superabsorbens mit Siliciumdioxid zu beschichten, aufweist.

22. Verfahren nach Anspruch 20 oder 21, **dadurch gekennzeichnet, dass** die schmierende Polyhydroxyverbindung Glycerol ist.

23. Verfahren nach Anspruch 20, 21 oder 22, **dadurch gekennzeichnet, dass** die relativen Mengen, in Masseteilen, in der Zubereitung
| | |
|---|---|
| für das Superabsorbens | 100 Masseteile, |
| für das Siliciumdioxid | 6 - 20 Masseteile, |
| für das Wasser | 0,5 - 10 Masseteile, |
| für die schmierende Polyhydroxyverbindung | 5 - 40 Masseteile |
betragen.

24. Flüssigkeitsabsorbierender Gegenstand mit einer Zubereitung, die in einem der Ansprüche 1 bis 21 definiert ist.

25. Gegenstand nach Anspruch 24, **dadurch gekennzeichnet, dass** der Gegenstand eine verdichtete Masse mit der Zubereitung aufweist.

26. Ein Stoma-, Inkontinenz- oder Wundversorgungsprodukt, das eine Superabsorbens-Zubereitung aufweist oder enthält, die in einem der Ansprüche 1 bis 19 definiert ist.

27. Produkt nach Anspruch 26, **dadurch gekennzeichnet, dass** das Produkt ein Stoma-Beutel ist.

28. Verwendung einer in einem der Ansprüche 1 bis 19 definierten Superabsorbens-Zubereitung in einem Stoma-, Inkontinenz- oder Wundversorgungsprodukt.

29. Verwendung nach Anspruch 28, **dadurch gekennzeichnet, dass** die Zubereitung in einer verdichteten Form als flüssigkeitsabsorbierender Gegenstand verwendet wird.

## Revendications

1. Formulation pour une utilisation afin d'absorber un liquide, comprenant :
un superabsorbant,
de la silice,
de l'eau, et
un composé polyhydroxy lubrifiant non volatil qui est soluble dans l'eau ou dispersible dans l'eau à 40 °C ou en dessous et est liquide à au moins une température se situant dans la plage de 15 à 20 °C,
**caractérisée en ce que** la quantité relative de la silice est d'au moins 6 parties en poids pour 100 parties en poids du superabsorbant.

2. Formulation selon la revendication 1, dans laquelle les quantités relatives dans la formulation sont en parties en poids :
| | |
|---|---|
| superabsorbant | 100 parties en poids |
| silice | 6 à 20 parties en poids |
| eau | 0,5 à 10 parties en poids |
| ledit composé polyhydroxy lubrifiant | 5 à 40 parties en poids. |

3. Formulation selon la revendication 1 ou 2, dans laquelle la silice est présente dans une quantité telle qu'elle fournit un revêtement de surface sur au moins la majorité de la superficie du superabsorbant.

4. Formulation selon la revendication 3, dans laquelle la silice est présente dans une quantité telle qu'elle fournit un revêtement de surface sur pratiquement toute la superficie du superabsorbant.

5. Formulation selon l'une quelconque des revendications précédentes, dans laquelle la silice est présente dans une quantité d'au moins 7 parties en poids.

6. Formulation selon la revendication 5, dans laquelle la silice est présente dans une quantité d'au moins 8 parties en poids.

7. Formulation selon l'une quelconque des revendications précédentes, dans laquelle le composé polyhydroxy est du glycérol.

8. Formulation selon l'une quelconque des revendications précédentes, dans laquelle le composé polyhydroxy est présent dans une quantité de 5 à 30 parties en poids.

9. Formulation selon l'une quelconque des revendications précédentes, dans laquelle le composé polyhydroxy est présent dans une quantité d'au moins 12 parties en poids.

10. Formulation selon la revendication 9, dans laquelle le composé polyhydroxy est présent dans une quantité d'au moins 13 parties en poids.

11. Formulation selon la revendication 10, dans laquelle le composé polyhydroxy est présent dans une quantité d'au moins 14 parties en poids.

12. Formulation selon l'une quelconque des revendications précédentes, dans laquelle l'eau est présente dans une quantité de 0,5 à 6 parties en poids.

13. Formulation selon l'une quelconque des revendications précédentes, dans laquelle l'eau est présente dans une quantité d'au moins 3 parties en poids.

14. Formulation selon la revendication 13, dans laquelle l'eau est présente dans une quantité d'au moins 4 parties en poids.

15. Formulation selon l'une quelconque des revendications précédentes, dans laquelle le superabsorbant comprend un polymère gonflable par l'eau.

16. Formulation selon la revendication 15, dans laquelle le superabsorbant comprend un polyacrylate.

17. Formulation selon la revendication 16, dans laquelle le polyacrylate est un polyacrylate de métal alcalin.

18. Formulation selon la revendication 17, dans laquelle le superabsorbant comprend du polyacrylate de sodium.

19. Formulation selon l'une quelconque des revendications précédentes, dans laquelle la formulation peut être consolidée lors d'une compression pour former une masse consolidée.

20. Procédé de production d'une formulation de superabsorbant, le procédé étant **caractérisé par** :
(a) la fourniture de particules de superabsorbant qui sont pratiquement toutes revêtues de silice dans une quantité d'au moins 6 parties en poids de silice pour 100 parties en poids du superabsorbant, et
(b) le mélange du superabsorbant revêtu de silice, d'eau et d'un composé polyhydroxy lubrifiant non volatil qui est soluble dans l'eau ou dispersible dans l'eau à 40 °C ou en dessous et qui est liquide à au moins une température se situant dans la plage de 15 °C à 20 °C.

21. Procédé selon la revendication 20, dans lequel l'étape (a) comprend l'étape secondaire consistant à :
mélanger ensemble le superabsorbant et la silice pour enduire le superabsorbant de silice.

22. Procédé selon la revendication 20 ou 21, dans lequel le composé polyhydroxy est du glycérol.

23. Procédé selon la revendication 20, 21 ou 22, dans lequel les quantités relatives dans la formulation sont en parties en poids :
| | |
|---|---|
| superabsorbant | 100 parties en poids |
| silice | 6 à 20 parties en poids |
| eau | 0,5 à 10 parties en poids |
| ledit composé polyhydroxy lubrifiant | 5 à 40 parties en poids. |

24. Article absorbant de liquide comprenant une formulation définie selon les revendications 1 à 21.

25. Article selon la revendication 24, dans lequel l'article comprend une masse consolidée comprenant ladite formulation.

26. Produit pour stomie, incontinence ou cicatrisation comprenant ou contenant une formulation de superabsorbant telle que définie dans l'une quelconque des revendications 1 à 19.

27. Produit selon la revendication 26, dans lequel le produit est une poche de stomie.

28. Utilisation d'une formulation de superabsorbant définie selon l'une quelconque des revendications 1 à 19 dans un produit pour stomie, incontinence ou cicatrisation.

29. Utilisation selon la revendication 28, dans laquelle ladite formulation est utilisée sous une forme consolidée en tant qu'article absorbant de liquide.
